(19) Europäisches Patentamt
European Patent Office
Office européen des brevets

(11) Publication number: **0 384 416 B1**

(12) **EUROPEAN PATENT SPECIFICATION**

(45) Date of publication of patent specification: **03.11.93**

(51) Int. Cl.[5]: **A61K 31/415**

(21) Application number: **90103279.7**

(22) Date of filing: **20.02.90**

The file contains technical information submitted after the application was filed and not included in this specification

# (54) A pharmaceutical composition containing hydantoin derivative.

(30) Priority: **22.02.89 JP 42743/89**
**10.03.89 JP 58694/89**
**07.02.90 JP 26139/90**

(43) Date of publication of application:
**29.08.90 Bulletin 90/35**

(45) Publication of the grant of the patent:
**03.11.93 Bulletin 93/44**

(84) Designated Contracting States:
**AT BE CH DE DK ES FR GB GR IT LI LU NL SE**

(56) References cited:
**EP-A- 0 193 415**
**EP-A- 0 258 476**
**EP-A- 0 264 586**
**FR-A- 2 455 046**
**US-A- 4 181 729**

**PATENT ABSTRACTS OF JAPAN vol. 7, no. 232 (C-190)(1377) 14 October 1983**

**PATENT ABSTRACTS OF JAPAN vol. 8, no. 56 (C-124)(1493) 14 March 1984**

(73) Proprietor: **SANWA KAGAKU KENKYUSHO CO., LTD.**
**No. 35, Higashi-sotobori-cho**
**Higashi-ku Nagoya-shi Aichi-ken(JP)**

(72) Inventor: **Kurono, Masayasu**
**35, Higashi-sotobori-cho**
**Higashi-ku, Nagoya, Aichi-ken(JP)**
Inventor: **Sato, Makoto**
**35, Higashi-sotobori-cho**
**Higashi-ku, Nagoya, Aichi-ken(JP)**
Inventor: **Yoshina, Shigeaki**
**35, Higashi-sotobori-cho**
**Higashi-ku, Nagoya, Aichi-ken(JP)**
Inventor: **Kondo, Yoshiya**
**35, Higashi-sotobori-cho**
**Higashi-ku, Nagoya, Aichi-ken(JP)**
Inventor: **Inoue, Tsuneaki**
**35, Higashi-sotobori-cho**
**Higashi-ku, Nagoya, Aichi-ken(JP)**
Inventor: **Inoue, Akiharu**
**35, Higashi-sotobori-cho**
**Higashi-ku, Nagoya, Aichi-ken(JP)**
Inventor: **Sawai, Kiichi**
**35, Higashi-sotobori-cho**
**Higashi-ku, Nagoya, Aichi-ken(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

(74) Representative: **Wächtershäuser, Günter, Dr.**
**Tal 29**
**D-80331 München (DE)**

## Description

The present invention relates to a pharmaceutical composition showing an inhibition to aldose reductase enzymes and a high absorption in vivo. The composition is administered to prevent and/or cure various complications due to diabetes, which are have been regarded as intractable diseases.

It has been known there may be caused due to diabetes specific chronic complications such as diabetic cataract, diabetic retinopathy, diabetic neuropathy, diabetic nephropathy and the like.

As one of studies for developing an effective agent to prevent and/or cure such diabetic complications, there is a search on inhibition substances to aldose reductase enzymes, since the enzymes reduce in vivo of human and other animals aldoses such as glucose and galactitose into corresponding polyols such as sorbitol and galactitol and it has been known that said complications will appear when the formed sorbitol and galactitol are accumulated at crystalline lens, peripheral nerve, kidney or the like in a patient with diabetes or galactosemia ["Jap. J. Opthamol.", Vol. 20, page 399 (1976), "Int. Congr. Ser. Excerpta Med.", Vol. 403, page 594 (1977) and "Metabolism", Vol. 28, page 456 (1979)].

The assignee company inclusive of the present inventors has also studied and investigated on inhibition substances to the aldose reductase enzymes. As a result, they have obtained the findings that racemic and optically active hydantoin derivatives shown by the following chemical formula show an excellent inhibition to the aldose reductase enzymes and effective for prevention and cure of the chronic and intractable complications due to diabetes, so that concerned patent applications have been filed [Jap. Pat. No. Sho 61 - 20091(A) (corresponding to USP 4740517 and EP-A-0 193 415) as well as Jap. Pat. Nos. Sho 63 - 57588-(A) and 63 - 126881(A) (corresponding to USP 4861792 and EP-A-0 264 586].

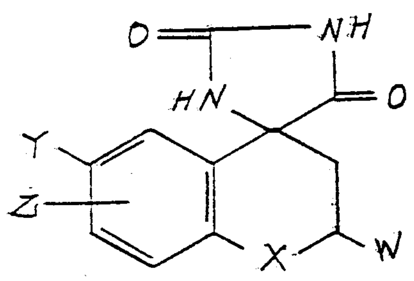

wherein W is halogenomethyl radical, 1H-tetrazol-5-yl radical, -COOR,

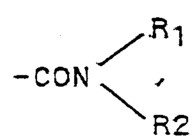

$-CH_2OR_3$ or

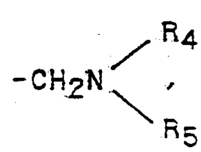

X is oxygen atom or sulfur atom, Y and Z are same or different and each represents hydrogen atom or halogen atom, R is alkyl group, alkoxy group or alkylmercapto group, $R_1$ and $R_2$ are same or different and each represents hydrogen atom, alkyl group, $-(CH_2CH_2O)_nCH_3$ or substituted phenyl group, or $R_1$ represents together with $R_2$ and the neighboring nitrogen atom or another nitrogen atom or oxygen atom a 5 or 6 membered heterocyclic ring, $R_3$ is hydrogen atom or alkyl group, $R_4$ and $R_5$ are same or different and each represents hydrogen atom or alkyl group, and n is an integer of 1 to 113.

The inventors have prepared in a conventional manner a pharmaceutical composition containing the hydantoin derivative as an effective ingredient for checking a solubility thereof in oral dosage and absorption by administering same to human to find that the solubility is not sufficient and absorption is about 40%, so that such composition shows relatively low biological availability.

In general, improvement in absorption and in biological availability of an effective ingredient are quite important for providing appearance of desired pharmacological action in constant and stable to lower undesirable side effects, so that concerned studies have energetically been made. As a result, various techniques have been developed, but there is no technique common to various agents or compounds, since the absorption in vivo and biological availability of the compounds are different depending on its kind.

It has been known in the art as one of general measures for solving the problems encountered on said hydantoin derivatives, namely the solubility and dissolving velocity thereof that an ionic or non-ionic surface active agent is composed. However, this measure is not preferable for the medicine in question, namely the hydantoin preparation, since the medicine shall be continuously administered for a long period of time to cure the chronic and intractable complication(s) due to diabetes and in such case, there is a fear of that the surface active agent composed in the medicine may cause a damage to mucosae in vivo.

A primary object of the invention is to provide a pharmaceutical composition containing the hydantoin derivative having an inhibition to aldose reductase enzymes and as an effective ingredient to prevent and cure various complications due to diabetes, said composition showing a good solubility in vivo, so that absorption and biological availability are remarkably improved.

A secondary object of the invention is to provide said pharmaceutical composition, wherein discharge of the hydantoin derivative as affective ingredient can be controlled in vivo.

According to the invention, the primary object can be attained by a pharmaceutical composition as defined in claim 1, wherein an optically active hydantoin derivative of the formula

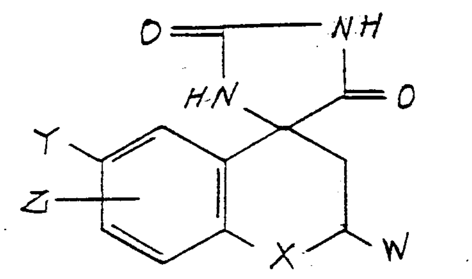

wherein W is halogenomethyl radical, 1H-tatrazol-5-yl radical, -COOR,

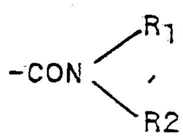

$-CH_2OR_3$ or

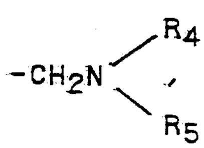

X is oxygen atom or sulfur atom, Y and Z are same or different and each represents hydrogen atom or halogen atom R is alkyl group, alkoxy groin or alkylmercapto group, $R_1$ and $R_2$ are same or different and each represents hydrogen atom, alkyl group, $-(CH_2CH_2O)_nCH_3$ or substituted phenyl group, or $R_1$ represents together with $R_2$ and the neighboring nitrogen atom or another nitrogen atom or oxygen atom a 5 or 6 membered heterocyclic ring, $R_3$ is hydrogen atom or alkyl group, $R_4$ and $R_5$ are same or different and each represents hydrogen atom or alkyl group, and n is an integer of 1 to 113,
is uniformly or homogeneously dispersed in a pharmacologically acceptable high molecular carrier as defined in claim 1 in an equi-amount by weight.

In this specification, the hydantoin derivative as the effective ingredient and shown by said formula may be referred to --remedy--, hereinafter.

Among the optically active hydantoin derivatives, followings may exemplary be listed.

a) d-6-Fluoro-2,3-dihydro-2',5'-dioxo-spiro[4H-1-benzopyran-4,4'-imidazolidine]-2-carboxamide,

b) d-2-Chloromethyl-6-fluoro-2,3-dihydro-spiro[4H-1-benzopyran-4,4'-imidazolidine]-2',5'-dione, and

c) d-2-Bromomethyl-6-fluoro-2,3-dihydro-spiro[4H-1-benzopyran-4,4'-imidazolidine]-2'5'-dione.

As the pharmacologically acceptable high molecular carrier to be used for the invention, there are refined sugar, sucrose, starch, cyclodextrin, sucrose fatty acid ester, pentitol, xylitol, hexitol, ethylene glycol, glycerin monostearate, cetanol, stearyl alcohol, galactosamine, glucosamine, caprylic acid ester, arginine, lysine caprylate, dodecylamine, undecylamine, hydroxypropylcellulose, hydroxypropylmethylcellulose, polyvinylpyrrolidone, polyacrylamide, polyethyleneglycol, protease inhibitor, trypsin inhibitor, gelatin, pepsin, albumin, globulin, protamine, casein, gum arabic, pluran, and glucomannan.

Such a pharmacologically acceptable high molecular carrier and the remedy, namely the hydantoin derivative as effective ingredient are composed and the remedy is uniformly or homogeneously dispersed in the carrier. The resulting precomposition shall be referred to as --modified remedy--hereinafter. The ratio of the the remedy and the carrier thereof for preparing the modified remedy defends on a kind of the carrier but, in general, 1 : 1in weight base. The pharmaceutical composition according to the invention shall be prepared by composing other ingredients to the modified remedy.

While, as the water-soluble high molecular substances for controlling a discharge of the remedy, hydroxypropycellulose, hydroxypropylmethlcellulose, carboxymethylcellulose, methylcellulose, ethylcellulose, pluran, polyvinylalcohol, glucomannan, gum arabic may be listed, although some of them are overlapped with the substances as listed before for the pharmacologically acceptable high molecular carrier. The water-soluble high molecular substances have characteristics of that it gradually absorb a digestive solution and more particularly gastric juice to cause a swell thereof and then gradually dissolves, when the remedy is formulated as orally dosing medicine. Therefore, it is preferable for controlling the discharge of the remedy in vivo to select at least one and more preferably two or more substances for combining the same, by taking specific gravity, swell, viscosity of the water-soluble high molecular substances into consideration.

The pharmaceutical composition according to the invention is, in general, orally dosed, so that it shall be formulated as tablets, granules, powder or capsules For formulating the composition into one of such type medicine, a vehicle, filler, glossing agent, binder, taste or smell modifier, disintegrator, coating agent, coloring agent and other additives accepted in the field of preparing medicines may be employed.

An amount of the remedy for human depends on king of the specific compound to be selected, kind of illness, age of a patient, period of time to be estimated for preventing or curing the disease and other various factors, but in case for an adult, it is preferable to give about 10 to 2000mg/day by, if necessary dividing same to 2 or 3 times/day.

The invention will now be further explained in more detail with reference to Examples for preparing cmpositions or medicines, Test Examples and Comparative Examples, which will refer to drawings, wherein

Fig. 1 is a graph showing results of that pharmaceutical composition according to the present invention obtained by Example 4 given later and and that according to a conventional manner obtained by Comparative Example 1 given later were orally administered to normal persons to check a transition of remedy concentration in blood;

Fig. 2 is a graph showing a change of remedy discharging rate of pharmacological compositions according to the invention obtained by Examples 6, 9 and 10 given later as well as that according to a conventional manner obtained by Comparative Example 2 given later; and

Fig. 3 is a graph showing results of that pharmaceutical compositions according to the present invention obtained by Example 6, 9 and 10 given later and that according to a conventional manner obtained by Comparative Example 2 given later were orally administered to Viegle dogs to check a transition or remedy concentration in blood.

Please note that in the followings, d-6-fluoro-2,3-dihydro-2′,5′-dioxo-spiro[4H-1-benzopyran-4,4′-imidazolidine]-2-carboxamide (hereinafter referred to as - -remedy A-- is solely employed as an effective ingredient of the hydantoin derivative, but a similar result has been obtained, when d-2-chloromethyl-6-fluoro-2,3-dihydro-spiro[4H-1-benzopyran-4,4′-imidazolidine]-2′,5′-dione or d-2-bromomethyl-6-fluoro-2,3-dihydro-spiro[4H-1-benzopyran-4,4′-imidazolidine]-2′,5′-dione is employed in lieu of said compound.

## Test Example 1

Each of refined sugar and albumin was selected as a pharmacologically acceptable high molecular carrier and was composed in various rate in weight with the remedy A to prepare test compositions.

To experimental animals of SD rats (weight of about 50g), the test composition was orally and forcedly administered at a dose of 0.4 - 5mg/kg once a day under giving a powdery diet containing galactose in

30%. Control animals were received the galactose diet containing no test composition. One day after final administration (on 9th day from the first administration), sciatic nerves were exentrated for galactitol determination to calculate 50% inhibition ($ED_{50}$ - based on the remedy A) in galactitol accumulation of test group to control group giving no test sample.

Results are shown in following Tables 1 and 2. As apparent, , it is preferable to select a composing rate of about 1 : 1, when the refined sugar or albumin is employed as the carrier (This homogenous composition of the remedy and carrier is the -modified remedy--).

Table 1

| Composing rate of refined sugar (%) | $ED_{50}$ (mg/kg) |
|---|---|
| 0 | 1.3 |
| 30 | 0.9 |
| 50 | 0.8 |
| 70 | 1.0 |
| 100 | - |

Table 2

| Composing rate of albumin (%) | $ED_{50}$ (mg/kg) |
|---|---|
| 0 | 1.3 |
| 30 | 1.0 |
| 50 | 0.9 |
| 70 | 1.1 |
| 100 | - |

Test Example 2 (Ratio of dissolving velocity constant in the remedy A)

a) Test sample

The remedy A was homogeneously mixed with or dissolved to various pharmacologically acceptable high molecular carriers in ratio of 1 : 9 or 1 : 1 to prepare test samples, said mixing or dissolving depending on physical properties of the substance selected as the carrier.

b) Testing method

According to "Discharging Test" in general testing methods teached in the Pharmacopoeia of Japan, 11th Edition.

Method :
The paddle method, 50rpm.
Amount of sample, volume :
50mg as the remedy A, 5ml.
Test solution for discharge, volume :
The 1st solution for disintegrating test, pH 1.2, 900ml.
Sampling period of time :
2, 4, 6, 8, 10, 20, 30, 60 and 120 minutes.

c) Quantitative method

Taking out the test solution by 5ml at each sampling time, measuring absorbance of the in the taken out solution at wavelength of 282nm, verifying the measured value to a pre-prepared calibration curve to know

an amount of the remedy A discharged into the solution from the test sample, calculating therefrom a constant of dissolving velocity in a conventional manner, and calculating a ratio of dissolving velocity constant to a constant of dissolving velocity of the remedy A per se, as control.

d) Result and consideration

Results are shown in following Tables 3 and 4. As apparent from the Tables, the dissolving velocity of the remedy A can be increased by changing a kind and amount of the high molecular carrier.

Table 3

| (remedy A : carrier = 1 : 9) | |
|---|---|
| Carrier | Ratio of dissolving velocity constant |
| Crystalline cellulose | 6.31 |
| HPC- L | 0.49 |
| PVPk-30 | 1.16 |
| Lactose | 2.30 |
| Sucrose | 5.74 |

Table 3

| (remedy A : carrier = 1 : 1, modified remedy A) | |
|---|---|
| Carrier | Ratio of dissolving velocity constant |
| Crystalline cellulose | 7.25 |
| Sucrose | 8.74 |
| Monoglyceride | 2.07 |
| Sucrose ester | 5.26 |

Example 1 (Tablet)

| Prescription : | |
|---|---|
| Remedy A | 30.0 (g) |
| Refined sugar | 30.0 |
| Crystalline cellulose | 15.6 |
| Carboxymethylcellulose (Ca salt) | 8.4 |
| Polyvinylpyrrolidone | 4.2 |
| Lactose | 30.6 |
| Magnesium stearate | 1.2 |
| | 120.0 (g) |

The remedy A was added to the refined sugar and homogeneously dispersed therein to prepare a modified remedy A. To the modified remedy A, the crystalline cellulose, carboxymethylcellulose (Ca salt), polyvinylpyrrolidone and lactose were added and mixed. After dried the mixture, the magnesium stearate was added to the dried mixture to mix throughout, and the resulting powdery mixture was conventionally treated to obtain tablets (each tablet contains therein 30mg of the remedy A).

Each of the tablets may be film coated in a conventional manner.

Example 2 (Tablet)

| Prescription : | |
|---|---|
| Remedy A | 30.0 (g) |
| Crystalline cellulose | 40.0 |
| Carboxymethylcellulose (Ca salt) | 8.4 |
| Lactose | 40.4 |
| Magnesium stearate | 1.2 |
| | 120.0 (g) |

The remedy A was added to 30.0g of the crystalline cellulose and homogeneously dispersed therein to prepare a modified remedy A. To the modified remedy A, remaining part of the crystalline cellulose, carboxymethylcellulose (Ca salt) and lactose were added and mixed. After dried the mixture, the magnesium stearate was added to the dried mixture to mix throughout, and the resulting powdery mixture was conventionally treated to obtain tablets (each tablet contains therein 30mg of the remedy A).

Each of the tablets may be film coated in a conventional manner.

Example 3 (Tablet)

| Prescription : | |
|---|---|
| Remedy A | 30.0 (g) |
| Glucose | 30.0 |
| Crystalline cellulose | 15.6 |
| Carboxymethylcellulose (Ca salt) | 8.4 |
| Corn starch | 12.0 |
| Lactose | 22.2 |
| Sodium laurylsulfate | 0.6 |
| Magnesium stearate | 1.2 |
| | 120.0 (g) |

The remedy A was added to the glucose and homogeneously dispersed therein to prepare a modified remedy A. To the modified remedy A, the crystalline cellulose, carboxymethylcellulose (Ca salt), corn starch, lactose and sodium laurylsulfate were added and mixed. After dried the mixture, the magnesium stearate was added to the dried mixture to mix throughout, and the resulting powdery mixture was conventionally treated to obtain tablets (each tablet contains therein 30mg of the remedy A).

Each of the tablets may be film coated in a conventional manner.

Example 4 (Tablet)

| Prescription : | |
|---|---|
| Remedy A | 30.0 (g) |
| Refined sugar | 30.0 |
| Crystalline cellulose | 15.6 |
| Carboxymethylcellulose (Ca salt) | 8.4 |
| Corn starch | 12.0 |
| Lactose | 23.4 |
| Magnesium stearate | 1.2 |
| | 120.0 (g) |

The remedy A was added to the refined sugar and homogeneously dispersed therein to prepare a modified remedy A. To the modified remedy A, the crystalline cellulose, carboxymethylcellulose (Ca salt),

corn starch and lactose were added and mixed. After dried the mixture, the magnesium stearate was added to the dried mixture to mix throughout, and the resulting powdery mixture was conventionally treated to obtain tablets (each tablet contains therein 30mg of the remedy A).

Each of the tablets may be film coated in a conventional manner.

Comparative Example 1 (Tablet)

| Prescription : | |
|---|---|
| Remedy A | 30.0 (g) |
| Crystalline cellulose | 15.6 |
| Carboxymethylcellulose (Ca salt) | 8.4 |
| Corn starch | 12.0 |
| Polyvinylpyrrolidone | 4.2 |
| Lactose | 48.6 |
| Magnesium stearate | 1.2 |
| | 120.0 (g) |

To the remedy A, the crystalline cellulose, carboxymethylcellulose (Ca salt), corn starch, polyvinylpyrrolidone and lactose were added and mixed. After dried the mixture, the magnesium stearate was added to the dried mixture to mix throughout, and the resulting powdery mixture was conventionally treated to obtain tablets (each tablet contains therein 30mg of the remedy A).

Each of the tablets may be film coated in a conventional manner.

Test Example 3 (Ratio of dissolving velocity constant of tablets)

A ratio of dissolving velocity constant in the remedy A of the tablets obtained by Examples 1 to 4 was checked in the manner same with that described in Test Example 1, but to a dissolving velocity constant in the remedy A of the tablet obtained by Comparative Example 1.

Results are shown in following Table 5. As apparent from the Table, the dissolving velocity can be controlled by suitably selecting a kind and amount of the high molecular carrier.

Table 5

| Tablet | Ratio of dissolving velocity constant |
|---|---|
| Example 1 | 4.85 |
| 2 | 4.23 |
| 3 | 2.91 |
| 4 | 5.23 |

Test Example 4 (Concentration of remedy in blood)

a) Test sample

Film coated tablets obtained by Example 4 and Comparative Example 1 were employed, as samples to be tested.

b) Testing method

Normal 6 human volunteers were classified into 2 groups. The tablet obtained by Example 4 was orally administered to the members in one of the groups and the tablet obtained by Comparative Example 1 was orally administered to the members in the other group. A blood letting was carried out at an interval for each member. The blood was centrifuged to obtain a blood plasma which was acidified with hydrochloric acid solution and fractionated by TLC. After treated with HPLC, an absorbance of the fraction was measured

at wavelength of 284nm and measured value was verified to a pre-prepared calibration curve to determine a concentration of the remedy in blood and then mean value of 3 members was calculated.

c) Result and consideration

Results are shown in Fig. 1. Areas under the curve ($AUC_{0-12\ hour}$) therein are as follows, which mean the composition (Example 4, prepared by once forming the modified remedy with use of the remedy and high molecular carrier, and then treating in a conventional manner) according to the invention sustains in higher level the remedy concentration in blood and increases its biological availability of 2 folds, in comparison with those of the conventional composition (Comparative Example 1, prepared by merely adding and mixing various ingredient inclusive of the remedy, without making the remedy into its modified state).

| | |
|---|---|
| Example 4 | 1020ng•h/ml |
| Comparative Example 1 | 612ng•h/ml |

Example 5 (Capsule)

| Prescription: | |
|---|---|
| Remedy A | 30.0 (g) |
| Glucose | 30.0 |
| Crystalline cellulose | 15.6 |
| Carboxymethylcellulose (Ca salt) | 8.4 |
| Corn starch | 12.0 |
| Lactose | 23.4 |
| Magnesium stearate | 0.6 |
| | 120.0 (g) |

The remedy A was added to the glucose and homogeneously dispersed therein through wetting and dissolution to prepare a modified remedy A. To the modified remedy A, the lactose, crystalline cellulose, carboxymethylcellulose (Ca salt), and corn starch were added and mixed. After dried the mixture, the magnesium stearate was added to the dried mixture to mix throughout, and the resulting powdery mixture was charged in gelatin capsules to obtain capsuled preparations (each capsule contains therein 30mg of the remedy A).

A ratio of dissolving velocity constant of the remedy on the capsule was 3.54 to the dissolving velocity constant on the film coated tablet obtained by Comparative Example 1.

Example 6 (Tablet)

| Prescription : | |
|---|---|
| Remedy A | 30.0 (g) |
| Glucose | 30.0 |
| Crystalline cellulose | 15.6 |
| Carboxymethylcellulose (Ca salt) | 8.4 |
| Corn starch | 12.0 |
| Lactose | 23.4 |
| Magnesium stearate | 0.6 |
| | 120.0 (g) |

The remedy A was added to the glucose and homogeneously dispersed therein to prepare a modified remedy A. To the modified remedy A, the crystalline cellulose, carboxymethylcellulose (Ca salt), corn starch and lactose were added and mixed. After dried the mixture, the magnesium stearate was added to the dried

mixture to mix throughout, and the resulting powdery mixture was conventionally treated to obtain tablets (each tablet contains therein 30mg of the remedy A).

Each of the tablets may be film coated in a conventional manner.

Example 7 (Tablet)

| Prescription : | |
|---|---|
| Remedy A | 30.0 (g) |
| Xylitol | 30.0 |
| Carboxymethylcellulose (Ca salt) | 8.4 |
| Crystalline cellulose | 15.6 |
| Corn starch | 12.0 |
| Lactose | 23.4 |
| Magnesium stearate | 0.6 |
| | 120.0 (g) |

The remedy A was added to the xylitol and homogeneously dispersed therein through wetting and dissolution thereof to prepare a modified remedy A. To the modified remedy A, the crystalline cellulose, carboxymethylcellulose (Ca salt), corn starch and lactose were added and mixed. After dried the mixture, the magnesium stearate was added to the dried mixture to mix throughout, and the resulting powdery mixture was conventionally treated to obtain tablets (each tablet contains therein 30mg of the remedy A).

Each of the tablets may be film coated in a conventional manner.

Example 8 (Tablet)

| Prescription : | |
|---|---|
| Remedy A | 30.0 (g) |
| Stearyl alcohol | 30.0 |
| Crystalline cellulose | 15.6 |
| Carboxymethylcellulose (Ca salt) | 8.4 |
| Corn starch | 12.0 |
| Lactose | 23.4 |
| Magnesium stearate | 0.6 |
| | 120.0 (g) |

The remedy A was added to the stearyl alcohol and homogeneously dispersed therein through wetting and dissolution thereof to prepare a modified remedy A. To the modified remedy A, the crystalline cellulose, carboxymethylcellulose (Ca salt), corn starch and lactose were added and mixed. After dried the mixture, the magnesium stearate was added to the dried mixture to mix throughout, and the resulting powdery mixture was conventionally treated to obtain tablets (each tablet contains therein 30mg of the remedy A).

Each of the tablets may be film coated in a conventional manner.

Example 9 (Capsule)

| Prescription : | |
|---|---|
| Remedy A | 30.0 (g) |
| Glycerin monostearate | 30.0 |
| Hydroxypropylmethylcellulose | 30.0 |
| Lactose | 29.4 |
| Magnesium stearate | 0.6 |
| | 120.0 (g) |

The remedy A was added to the glycerin monostearate and homogeneously dispersed therein to prepare a modified remedy A. To the modified remedy A, the hydroxypropylmethylcellulose and lactose were added and mixed. After dried the mixture, the magnesium stearate was added to the dried mixture to mix throughout, and the resulting powdery mixture was charged in gelatin capsules to obtain capsuled preparations (each capsule contains therein 30mg of the remedy A).

Example 10 (Capsule)

| Prescription : | |
|---|---|
| Remedy A | 30.0 (g) |
| Hydroxypropylmethylcellulose | 30.0 |
| Lactose | 59.4 |
| Magnesium stearate | 0.6 |
| | 120.0 (g) |

The remedy A was added to the hydroxypropylmethylcellulose and homogeneously dispersed therein to prepare a modified remedy A. To the modified remedy A, the lactose was added and mixed. After dried the mixture, the magnesium stearate was added to the dried mixture to mix throughout, and the resulting powdery mixture was charged in gelatin capsules to obtain capsuled preparations (each capsule contains therein 30mg of the remedy A).

Example 11 (Capsule)

| Prescription : | |
|---|---|
| Remedy A | 30.0 (g) |
| Xylitol | 30.0 |
| Carboxymethylcellulose | 30.0 |
| Lactose | 29.4 |
| Magnesium stearate | 0.6 |
| | 120.0 (g) |

The remedy A was added to the xylitol and homogeneously dispersed therein through wetting and dissolution thereof to prepare a modified remedy A. To the modified remedy A, the carboxymethylcellulose and lactose were added and mixed. After dried the mixture, the magnesium stearate was added to the dried mixture to mix throughout, and the resulting powdery mixture was charged in gelatin capsules to obtain capsuled preparations (each capsule contains therein 30mg of the remedy A).

Example 12 (Capsule)

| Prescription : | |
|---|---|
| Remedy A | 30.0 (g) |
| Glucose | 30.0 |
| Hydroxypropylmethylcellulose | 30.0 |
| Lactose | 29.4 |
| Magnesium stearate | 0.6 |
| | 120.0 (g) |

The remedy A was added to the glucose and homogeneously dispersed therein to prepare a modified remedy A. To the modified remedy A, the hydroxypropylmethylcellulose and lactose were added and mixed. After dried the mixture, the magnesium stearate was added to the dried mixture to mix throughout, and the resulting powdery mixture was charged in gelatin capsules to obtain capsuled preparations (each capsule contains therein 30mg of the remedy A).

Example 13 (Capsule)

| Prescription : | |
|---|---|
| Remedy A | 30.0 (g) |
| Glucosamine | 30.0 |
| Pluran | 30.0 |
| Lactose | 29.4 |
| Magnesium stearate | 0.6 |
| | 120.0 (g) |

The remedy A was added to the glucosamine and homogeneously dispersed therein to prepare a modified remedy A. To the modified remedy A, the pluran and lactose were added and mixed. After dried the mixture, the magnesium stearate was added to the dried mixture to mix throughout, and the resulting powdery mixture was charged in gelatin capsules to obtain capsuled preparations (each capsule contains therein 30mg of the remedy A).

Example 14 (Capsule)

| Prescription : | |
|---|---|
| Remedy A | 30.0 (g) |
| Dodecylamine | 30.0 |
| Pluran | 30.0 |
| Crystalline cellulose | 15.0 |
| Lactose | 14.4 |
| Magnesium stearate | 0.6 |
| | 120.0 (g) |

The remedy A was added to the dodecylamine and homogeneously dispersed therein through wetting and dissolution thereof to prepare a modified remedy A. To the modified remedy A, the pluran, crystalline cellulose and lactose were added and mixed. After dried the mixture, the magnesium stearate was added to the dried mixture to mix throughout, and the resulting powdery mixture was charged in gelatin capsules to obtain capsuled preparations (each capsule contains therein 30mg of the remedy A).

Comparative Example 2 (Tablet)

| Prescription : | |
|---|---|
| Remedy A | 30.0 (g) |
| Hydroxypropylcellulose | 3.0 |
| Carboxymethylcellulose (Ca salt) | 10.0 |
| Crystalline cellulose | 6.4 |
| Lactose | 70.0 |
| Magnesium stearate | 0.6 |
| | 120.0 (g) |

To the remedy A, the crystalline cellulose, carboxymethylcellulose (Ca salt), hydroxypropylcellulose and lactose were added and mixed. After dried the mixture, the magnesium stearate was added to the dried mixture to mix throughout, and the resulting powdery mixture was conventionally treated to obtain tablets (each tablet contains therein 30mg of the remedy A).

Each of the tablets may be film coated in a conventional manner.

Test Example 5 (Test on discharge of remedy A from medicine preparation)

a) Test sample

The tablet obtained by Example 9 and capsules obtained by Examples 9 and 10 were selected as Test Samples and the tablet obtained by Comparative Example 2 was selected as Control Sample.

b) Testing method

According to "Discharging Test" in general testing methods teached in the Pharmacopoeia of Japan, 11th Edition.

Method :
The paddle method, 50rpm.

Amount of sample, volume :
50mg as the remedy A, 5ml.

Test solution for discharge, volume :
The 1st solution for disintegrating test, pH 1.2, 900ml.

Sampling period of time :
2, 4, 6, 8, 10, 20, 30, 60, 70, 80 and 90 minutes.

c) Quantitative method

Taking out the test solution by 5ml at each sampling time, measuring absorbance of the the taken-out solution at wavelength of 282nm, verifying the measured value to a pre-prepared calibration curve to determine an amount of the remedy A discharged into the solution from the test sample, and calculating a discharging rate of the remedy A.

d) Results and consideration

Results are shown in Fig. 2. There is seen such a tendency that the discharging rate of the pharmaceutical compositions (Examples 9 and 10) according to the invention is more slow and thus the remedy is gradually absorbed through digestive tubes, in comparison with the conventional composition (Comparative Example 2). While, the composition (Example 6) among those according to the invention shows rapid discharge of the remedy A, in comparison with the discharging rate of the conventional composition (Comparative Example 2), so that an immediate effect can be expected on the composition (Example 6).

Therefore, there is a possibility to provide a medicine with immediate appearance and continuation of its pharmacological effect.

Test Example 6 (Absorbing test in vivo)

a) Sample

Test Samples :

Tablet (Example 6),
Capsules (Examples 9 and 10).

Control Sample :

Tablet (Comparative Example 2).

b) Testing method

A concentration of the remedy A in blood was checked in accordance with the manner described in Test Example 4, except that Viegle dogs being fested for one overnight (weight of about 10kg, 3 heads in each group) were employed as experimental animals.

c) Result and consideration

Results are shown in following Table 6 and Fig. 3. As apparent therefrom, the remedy concentration in blood can be maintained in high level and the biological availability of the remedy becomes about 2 folds, in comparison with the conventional composition, when the pharmaceutical composition according to the invention is administered.

Table 6

| Sample | AUC 0-12 hour | |
|---|---|---|
| | ng• h /ml | ratio |
| Test Sample | | |
| Example 6 | 10600 | 1.6 |
| 9 | 15910 | 2.4 |
| 10 | 11900 | 1.8 |
| Control Sample | | |
| Comparative Example 2 | 6620 | 1.0 |

## Claims

**Claims for the following Contracting States : AT, BE, CH, DE, DK, FR, GB, IT, LI, LU, NL, SE**

1. A pharmaceutical composition for dosage, which shows an inhibition to aldose reductase enzymes, a good solubility in vivo and an increased biological availability, characterized in that said composition is obtained through the steps of homogenously dispersing an optically active hydantoin compound of the formula

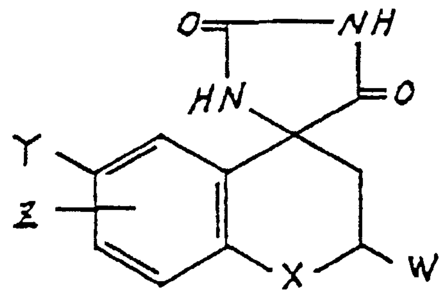

wherein W is halogenomethyl radical, 1H-tetrazol-5-yl radical, -COOR,

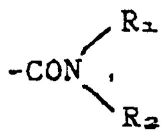

$-CH_2OR_3$, or

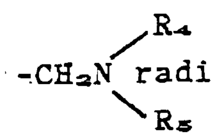 radical.

X is oxygen atom or sulfur atom, Y and Z are same or different and each represents hydrogen atom or halogen atom, R is alkyl group, alkoxy group or alkylmercapto group, $R_1$ and $R_2$ are same or different and each represents hydrogen atom, alkyl group, $-(CH_2CH_2O)_nCH_3$ or substituted phenyl group, or $R_1$ represents together with $R_2$ and the neighbouring nitrogen atom, another nitrogen atom or oxygen atom a 5 or 6 membered heterocyclic ring, $R_3$ is hydrogen atom or alkyl group, $R_4$ and $R_5$ are same or different and each represents hydrogen atom or alkyl group, and $_n$ is an integer of 1 to 113,
into a pharmacologically acceptable high molecular substance in an equi-amount by weight and selected from sugar, sucrose, glucose, starch, cyclodextrin, sucrose fatty acid ester, pentitol, xylitol, hexitol, ethylene glycol, glycerin monostearate, cetanol, stearyl alcohol, galactosamine, glucosamine, caprylic acid ester, arginine, lysine, caprylate, dodecylamine, undecylamine, hydroxypropylcellulose, hydroxypropylmethylcellulose, polyvinylpyrrolidone, polyacryloamide, polyethylene glycol, protease inhibitor, trypsine inhibitor, gelatin, pepsin, albumin, globulin, protamine, casein, gum arbic, pluran and glucomannan to prepare a precomposition: mixing the pre-composition with a conventional pharmacologically acceptable carrier; and treating the resulting mixture in a conventional manner to prepare the pharmaceutical composition.

**2.** A pharmaceutical composition as claimed in Claim 1, characterized in that before the treatment with the conventional pharmacologically acceptable carrier, said pre-composition is mixed with a water-soluble high molecular substance selected from hydroxypropylcellulose, hydroxypropylmethylcellulose, carboxymethylcellulose, methylcellulose, ethylcellulose, pluran, polyvinylalcohol, glucomannan and gum arabic, to control a discharge of the hydantoin compound in vivo.

**3.** A pharmaceutical composition as claimed in Claim 1 or 2, characterized in that said hydantoin compound is at least one of those selected from
a) d-6-fluoro-2,3-dihydro-2',5'-dioxo-spiro[4H-1-benzopyran-4-4'imidazolidine]-2-carboxamide.
b) d-2-chloromethyl-6-fluoro-2,3-dihydro-spiro[4H-1-benzopyran-4,4'imidazolidine]-2',5'-dione, and
c) d-2-bromomethyl-6-fluoro-2,3-dihydro-spiro[4H-1-benzopyran-4,4'imidazolidine]-2',5'-dione.

**Claims for the following Contracting States : ES, GR**

1. A method for preparing a pharmaceutical composition for dosage, which shows an inhibition to aldose reductase enzymes, a good solubility in vivo and an increased biological availability, characterized in that said method comprises the steps of homogenously dispersing an optically active hydantoin compound of the formula

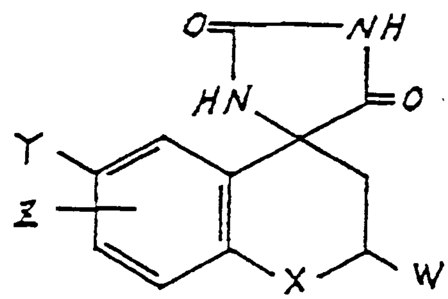

wherein W is halogenomethyl radical, 1H-tetrazol-5-yl radical, -COOR,

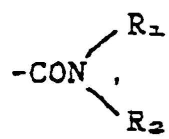

,

$-CH_2OR_3$, or

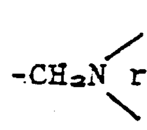

radical.

X is oxygen atom or sulfur atom, Y and Z are same or different and each represents hydrogen atom or halogen atom, R is alkyl group, alkoxy group or alkylmercapto group, $R_1$ and $R_2$ are same or different and each represents hydrogen atom, alkyl group, $-(CH_2CH_2O)_nCH_3$ or substituted phenyl group, or $R_1$ represents together with $R_2$ and the neighbouring nitrogen atom, another nitrogen atom or oxygen atom a 5 or 6 membered heterocyclic ring, $R_3$ is hydrogen atom or alkyl group, $R_4$ and $R_5$ are same or different and each represents hydrogen atom or alkyl group, and $_n$ is an integer of 1 to 113,
into a pharmacologically acceptable high molecular substance in an equi-amount by weight and selected from sugar, sucrose, glucose, starch, cyclodextrin, sucrose fatty acid ester, pentitol, xylitol, hexitol, ethylene glycol, glycerin monostearate, cetanol, stearyl alcohol, galactosamine, glucosamine, caprylic acid ester, arginine, lysine, caprylate, dodecylamine, undecylamine, hydroxypropylcellulose, hydroxypropylmethycellulose, polyvinylpyrrolidone, polyacryloamide, polyethylene glycol, protease inhibitor, trypsine inhibitor, gelatin, pepsin, albumin, globulin, protamine, casein, gum arbic, pluran and glucomannan to prepare a pre-composition: mixing the pre-composition with a conventional pharmacologically acceptable carrier; and treating the resulting mixture in a conventional manner to prepare the pharmaceutical composition.

2. A method as claimed in Claim 1, characterized in that before the treatment with the conventional pharmacologically acceptable carrier, said pre-composition is mixed with a water-soluble high molecular substance selected from hydroxypropylcellulose, hydroxypropylmethylcellulose, carboxymethylcellulose, methylcellulose, ethylcellulose, pluran, polyvinylalcohol, glucomannan and gum arabic, to control a discharge of the hydantoin compound in vivo.

3. A method as claimed in Claim 1 or 2, characterized in that said hydantoin compound is at least one of those selected from

a) d-6-fluoro-2,3-dihydro-2',5'-dioxo-spiro[4H-1-benzopyran-4,4'imidazolidine]-2-carboxamide,
b) d-2-chloromethyl-6-fluoro-2,3-dihydro-spiro[4H-1-benzopyran-4,4'imidazolidine]-2',5'-dione, and
c) d-2-bromomethyl-6-fluoro-2,3-dihydro-spiro[4H-1-benzopyran-4,4'imidazolidine]-2',5'-dione.

## Patentansprüche

**Patentansprüche für folgende Vertragsstaaten : AT, BE, CH, DE, DK, FR, GB, IT, LI, LU, NL, SE**

**1.** Arzneimittel zur Dosierung, das eine Hemmung für Aldosereduktaseenzyme, eine gute Löslichkeit in vivo und eine erhöhte biologische Verfügbarkeit aufweist, **dadurch gekennzeichnet**, daß die Zusammensetzung erhalten wird durch die Schritte der homogenen Dispersion einer optisch aktiven Hydantoinverbindung der Formel

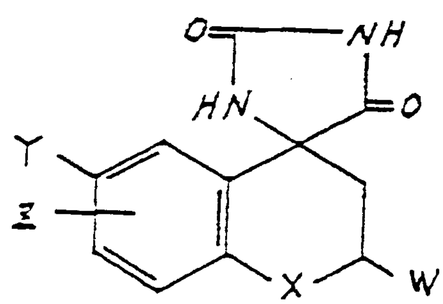

worin W einen Halogenmethylrest, 1H-Tetrazol-5-ylrest, -COOR,

$$-CON(R_1)(R_2),$$

$-CH_2OR_3$ oder

$$-CH_2N(R_4)(R_5)$$ bedeutet,

X ein Sauerstoffatom oder Schwefelatom darstellt, Y und Z gleich oder verschieden sind und jeweils ein Wasserstoffatom oder Halogenatom darstellen, R eine Alkylgruppe, Alkoxygruppe oder Alkylmercaptogruppe bedeutet, $R_1$ und $R_2$ gleich oder verschieden jeweils ein Wasserstoffatom, eine Alkylgruppe, $-(CH_2CH_2O)_nCH_3$ oder eine substituierte Phenylgruppe bedeuten oder $R_1$ zusammen mit $R_2$ und dem benachbarten Stickstoffatom, einem anderen Stickstoffatom oder Sauerstoffatom einen 5- oder 6-gliedrigen heterocyclischen Ring bilden, $R_3$ ein Wasserstoffatom oder eine Alkylgruppe bedeutet, $R_4$ und $R_5$ gleich oder verschieden jeweils ein Wasserstoffatom oder eine Alkylgruppe darstellen und n eine ganze Zahl von 1 bis 113 bedeutet, in eine pharmakologisch verträgliche hochmolekulare Substanz in einer äquimolaren Gewichtsmenge und ausgewählt aus Zucker, Saccharose, Glucose, Stärke, Cyclodextrin, Saccharosefettsäureester, Pentit, Xylit, Hexit, Ethylenglycol, Glycerinmonostearat, Cetanol, Stearylalkohol, Galactosamin, Glucosamin, Caprylsäureester, Arginin, Lysin, Caprylat, Dodecylamin, Undecylamin, Hydroxypropylcellulose, Hydroxypropylmethylcellulose, Polyvinylpyrrolidon, Polyacrylamid, Polyethylenglycol, Proteaseinhibitor, Trypsininhibitor, Gelatine, Pepsin, Albumin, Globulin, Protamin, Casein, Gummi Arabicum, Pluran und Glucomannan unter Herstellung einer Vorzusammensetzung: Mischen der Vorzusammensetzung mit einem üblichen pharmakologisch verträglichen Träger und Behandeln des erhaltenen Gemisches in üblicher Weise unter Herstellung des Arzneimittels.

2. Arzneimittel nach Anspruch 1, **dadurch gekennzeichnet**, daß vor der Behandlung mit dem üblichen pharmakologisch verträglichen Träger die Vorzusammensetzung mit einer wasserlöslichen hochmolekularen Substanz, ausgewählt aus Hydroxypropylcellulose, Hydroxypropylmethylcellulose, Carboxymethylcellulose, Methylcellulose, Ethylcellulose, Pluran, Polyvinylalkohol, Glucomannan und Gummi Arabicum vermischt wird, um die Abgabe der Hydantoinverbindung in vivo zu steuern.

3. Arzneimittel nach Anspruch 1 oder 2, **dadurch gekennzeichnet**, daß die Hydantoinverbindung mindestens eine ausgewählt aus
   a) d-6-Fluor-2,3-dihydro-2',5'-dioxo-spiro[4H-1-benzopyran-4,4'-imidazolidin]-2-carboxamid,
   b) d-2-Chlormethyl-6-fluor-2,3-dihydro-spiro[4H-1-benzopyran-4,4'-imidazolidin]-2',5'-dion und
   c) d-2-Brommethyl-6-fluor-2,3-dihydro-spiro[4H-1-benzopyran-4,4'-imidazolidin]-2',5'-dion ist.

**Patentansprüche für folgende Vertragsstaaten : ES, GR**

1. Verfahren zur Herstellung eines Arzneimittels zur Dosierung, das eine Hemmung für Aldosereduktaseenzyme, eine gute Löslichkeit in vivo und eine erhöhte biologische Verfügbarkeit aufweist, dadurch gekennzeichnet, daß die Zusammensetzung erhalten wird durch die Schritte der homogenen Dispersion einer optisch aktiven Hydantoinverbindung der Formel

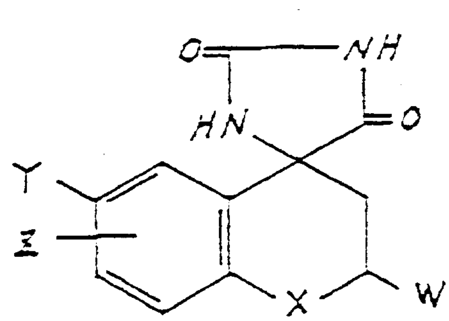

worin W einen Halogenmethylrest, 1H-Tetrazol-5-ylrest, -COOR,

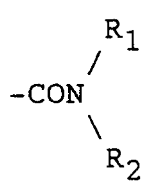 ,

$-CH_2OR_3$ oder

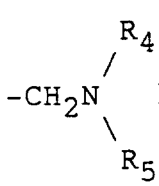 bedeutet,

X ein Sauerstoffatom oder Schwefelatom darstellt, Y und Z gleich oder verschieden sind und jeweils ein Wasserstoffatom oder Halogenatom darstellen, R eine Alkylgruppe, Alkoxygruppe oder Alkylmercaptogruppe bedeutet, $R_1$ und $R_2$ gleich oder verschieden jeweils ein Wasserstoffatom, eine Alkylgruppe, $-(CH_2CH_2O)_nCH_3$ oder eine substituierte Phenylgruppe bedeuten oder $R_1$ zusammen mit $R_2$ und dem benachbarten Stickstoffatom, einem anderen Stickstoffatom oder Sauerstoffatom einen 5- oder 6-gliedrigen heterocy-

clischen Ring bilden, $R_3$ ein Wasserstoffatom oder eine Alkylgruppe bedeutet, $R_4$ und $R_5$ gleich oder verschieden jeweils ein Wasserstoffatom oder eine Alkylgruppe darstellen und n eine ganze Zahl von 1 bis 113 bedeutet, in eine pharmakologisch verträgliche hochmolekulare Substanz in einer äquimolaren Gewichtsmenge und ausgewählt aus Zucker, Saccharose, Glucose, Stärke, Cyclodextrin, Saccharosefettsäureester, Pentit, Xylit, Hexit, Ethylenglycol, Glycerinmonostearat, Cetanol, Stearylalkohol, Galactosamin, Glucosamin, Caprylsäureester, Arginin, Lysin, Caprylat, Dodecylamin, Undecylamin, Hydroxypropylcellulose, Hydroxypropylmethylcellulose, Polyvinylpyrrolidon, Polyacrylamid, Ethylenglycol, Proteaseinhibitor, Trypsininhibitor, Gelatine, Pepsin, Albumin, Globulin, Protamin, Casein, Gummi Arabicum, Pluran und Glucomannan unter Herstellung einer Vorzusammensetzung: Mischen der Vorzusammensetzung mit einem üblichen pharmakologisch verträglichen Träger und Behandeln des erhaltenen Gemisches in üblicher Weise unter Herstellung des Arzneimittels.

2. Verfahren nach Anspruch 1, **dadurch gekennzeichnet**, daß vor der Behandlung mit dem üblichen pharmakologisch verträglichen Träger die Vorzusammensetzung mit einer wasserlöslichen hochmolekularen Substanz, ausgewählt aus Hydroxypropylcellulose, Hydroxypropylmethylcellulose, Carboxymethylcellulose, Methylcellulose, Ethylcellulose, Pluran, Polyvinylalkohol, Glucomannan und Gummi Arabicum vermischt wird, um die Abgabe der Hydantoinverbindung in vivo zu steuern.

3. Verfahren nach Anspruch 1 oder 2, **dadurch gekennzeichnet**, daß die Hydantoinverbindung mindestens eine darstellt ausgewählt aus
   a) d-6-Fluor-2,3-dihydro-2',5'-dioxo-spiro[4H-1-benzopyran-4,4'-imidazolidin]-2-carboxamid,
   b) d-2-Chlormethyl-6-fluor-2,3-dihydro-spiro[4H-1-benzopyran-4,4'-imidazolidin]-2',5'-dion und
   c) d-2-Brommethyl-6-fluor-2,3-dihydro-spiro[4H-1-benzopyran-4,4'-imidazolidin]-2',5'-dion ist.

## Revendications

**Revendications pour les Etats contractants suivants : AT, BE, CH, DE, DK, FR, GB, IT, LI, LU, NL, SE**

1. Composition pharmaceutique pour administration, qui inhibe les enzymes aldose réductase et fait preuve d'une bonne solubilité in vivo et d'une biodisponibilité accrue, caractérisée en ce que l'on obtient ladite composition par mise en oeuvre des étapes consistant
   - à disperser de façon homogène un composé d'hydantoïne optiquement actif de formule

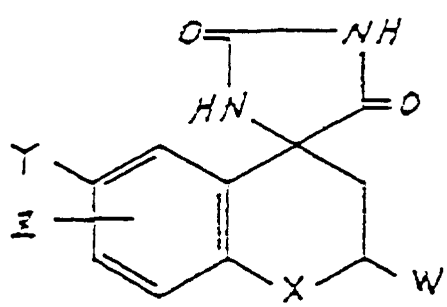

dans laquelle W est un radical halogénométhyle, un radical 1 H-tétrazol-5-yle, un radical

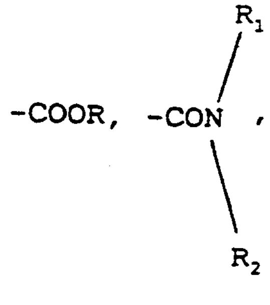

$-CH_2OR_3$ ou

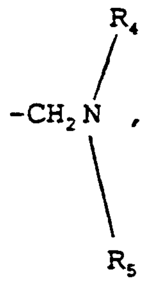

X est un atome d'oxygène ou un atome de soufre, Y et Z sont identiques ou différents et chacun représente un atome d'hydrogène ou un atome halogène, R est un groupe alkyle, un groupe alkoxy ou un groupe alkylmercapto, $R_1$ et $R_2$ sont identiques ou différents, et chacun représente un atome d'hydrogène, un groupe alkyle, un groupe $-(CH_2CH_2O)_nCH_3$ ou un groupe phényle substitué, ou bien $R_1$ forme avec $R_2$ et l'atome d'azote voisin, un autre atome d'azote ou un atome d'oxygène, un hétérocycle à 5 ou 6 chaînons, $R_3$ est un atome d'hydrogène ou un groupe alkyle, $R_4$ et $R_5$ sont identiques ou différents et chacun représente un atome d'hydrogène ou un groupe alkyle, et n est un nombre entier de 1 à 113, dans une substance de poids moléculaire élevé pharmacologiquement acceptable en quantité égale en poids et choisie parmi un sucre, le saccharose, le glucose, l'amidon, la cyclodextrine, l'ester d'acide gras et de saccharose, le pentitol, le xylitol, l'hexitol, l'éthylène glycol, le monostéarate de glycérine, le cétanol, l'alcool stéarylique, la galactosamine, la glucosamine, l'ester de l'acide caprylique, l'arginine, la lysine, le caprylate, la dodécylamine, l'undécylamine, l'hydroxypropylcellulose, l'hydroxypropylméthylcellulose, la polyvinylpyrrolidone, le polyacrylamide, le polyéthylène glycol, l'inhibiteur de la protéase, l'inhibiteur de la trypsine, la gélatine, la pepsine, l'albumine, la globuline, la protamine, la caséine, la gomme arabique, le plurane et le glucomannane, pour préparer une pré-composition;

- à mélanger la pré-composition avec un support conventionnel pharmacologiquement acceptable; et
- à traiter de façon conventionnelle le mélange résultant afin de préparer la composition pharmaceutique.

**2.** Composition pharmaceutique comme revendiquée dans la revendication 1, caractérisée en ce que, avant le traitement par le support conventionnel pharmacologiquement acceptable, ladite pré-composition est mélangée avec une substance soluble dans l'eau et de poids moléculaire élevé, choisie parmi l'hydroxypropylcellulose, l'hydroxypropylméthylcellulose, la carboxyméthylcellulose, la méthylcellulose, l'éthylcellulose, le plurane, l'alcool polyvinylique, le glucomannane et la gomme arabique, pour contrôler la libération in vivo du composé d'hydantoïne.

**3.** Composition pharmaceutique comme revendiquée dans la revendication 1 ou 2, caractérisée en ce que ledit composé d'hydantoïne est au moins l'un de ceux choisis parmi

a) le d-6-fluoro-2,3-dihydro-2',5'-dioxo-spiro-[4H-1-benzo-pyranne-4,4'imidazolidine]-2-carboxamide,
b) la d-2-chlorométhyl-6-fluoro-2,3-dihydro-spiro-[4H-1-benzo-pyranne-4,4'imidazolidine]-2',5'-dione, et
c) la d-2-bromométhyl-6-fluoro-2,3-dihydro-spiro-[4H-1-benzopyranne-4,4'imidazolidine]-2',5'-dione.

**Revendications pour les Etats contractants suivants : ES, GR**

**1.** Procédé pour préparer une composition pharmaceutique pour administration, qui inhibe les enzymes aldose réductase et fait preuve d'une bonne solubilité in vivo et d'une biodisponibilité accrue, caractérisé en ce que ledit procédé comprend les étapes consistant

- à disperser de façon homogène un composé d'hydantoïne optiquement actif de formule

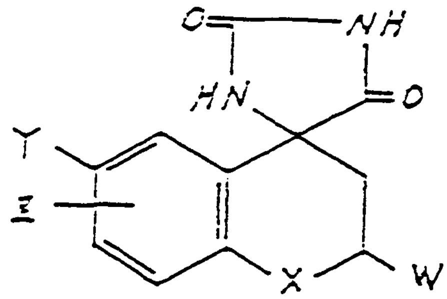

dans laquelle W est un radical halogénométhyle, un radical 1 H-tétrazol-5-yle, un radical

$-COOR$, 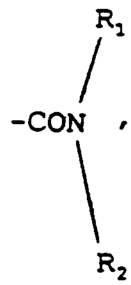

$-CH_2OR_3$ ou

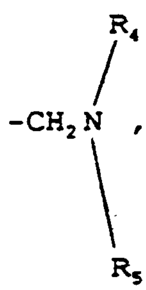

X est un atome d'oxygène ou un atome de soufre, Y et Z sont identiques ou différents et chacun représente un atome d'hydrogène ou un atome halogène, R est un groupe alkyle, un groupe alkoxy ou un groupe alkylmercapto, $R_1$ et $R_2$ sont identiques ou différents et chacun représente un atome d'hydrogène, un groupe alkyle, un groupe $-(CH_2CH_2O)_nCH_3$ ou un groupe phényle substitué, ou bien $R_1$ forme avec $R_2$ et l'atome d'azote voisin, un autre atome d'azote ou un atome d'oxygène, un hétérocycle à 5 ou 6 chaînons, $R_3$ est un atome d'hydrogène ou un groupe alkyle, $R_4$ et $R_5$ sont identiques ou différents et chacun représente un atome d'hydrogène ou un groupe alkyle, et n est un nombre entier de 1 à 113,
dans une substance de poids moléculaire élevé pharmacologiquement acceptable en quantité égale en poids set choisie parmi le sucre, le saccharose, le glucose, l'amidon, la cyclodextrine, l'ester d'acide gras et de saccharose, le pentitol, le xylitol, l'hexitol, l'éthylène glycol, le monostéarate de glycérine, le cétanol, l'alcool stéarylique, la galactosamine, la glucosamine, l'ester de l'acide caprylique, l'arginine, la lysine, le caprylate, la dodécylamine, l'undécylamine, l'hydroxypropylcellulose, l'hydroxypropylméthylcellulose, la polyvinylpyrrolidone, le polyacrylamide, le polyéthylène glycol, l'inhibiteur de la protéase, l'inhibiteur de la trypsine, la gélatine, la pepsine, l'albumine, la globuline, la protamine, la caséine, la gomme arabique, le plurane et le glucomannane, pour préparer une pré-composition;
- à mélanger la pré-composition avec un support conventionnel pharmacologiquement acceptable; et

- à traiter de façon conventionnelle le mélange résultant afin de préparer la composition pharmaceutique.

**2.** Procédé comme revendiqué dans la revendication 1, caractérisé en ce que, avant le traitement par le support conventionnel pharmacologiquement acceptable, ladite précomposition est mélangée avec une substance soluble dans l'eau et de poids moléculaire élevé, choisie parmi l'hydroxypropylcellulose, l'hydroxypropylméthylcellulose, la carboxyméthylcellulose, la méthylcellulose, l'éthylcellulose, le plurane, l'alcool polyvinylique, le glucomannane, et la gomme arabique, pour contrôler la libération in vivo du composé d'hydantoïne.

**3.** Procédé comme revendiqué dans la revendication 1 ou 2, caractérisé en ce que ledit composé d'hydantoïne est au moins l'un de ceux choisis parmi

a) le d-6-fluoro-2,3-dihydro-2',5'-dioxo-spiro-[4H-1-benzo-pyranne-4,4'imidazolidine]-2-carboxamide,

b) la d-2-chlorométhyl-6-fluoro-2,3-dihydro-spiro-[4H-1-benzo-pyranne-4,4'imidazolidine]-2',5'-dione, et

c) la d-2-bromométhyl-6-fluoro-2,3-dihydro-spiro-[4H-1-benzo-pyranne-4,4'imidazolidine]-2',5'-dione.

FIG. 1

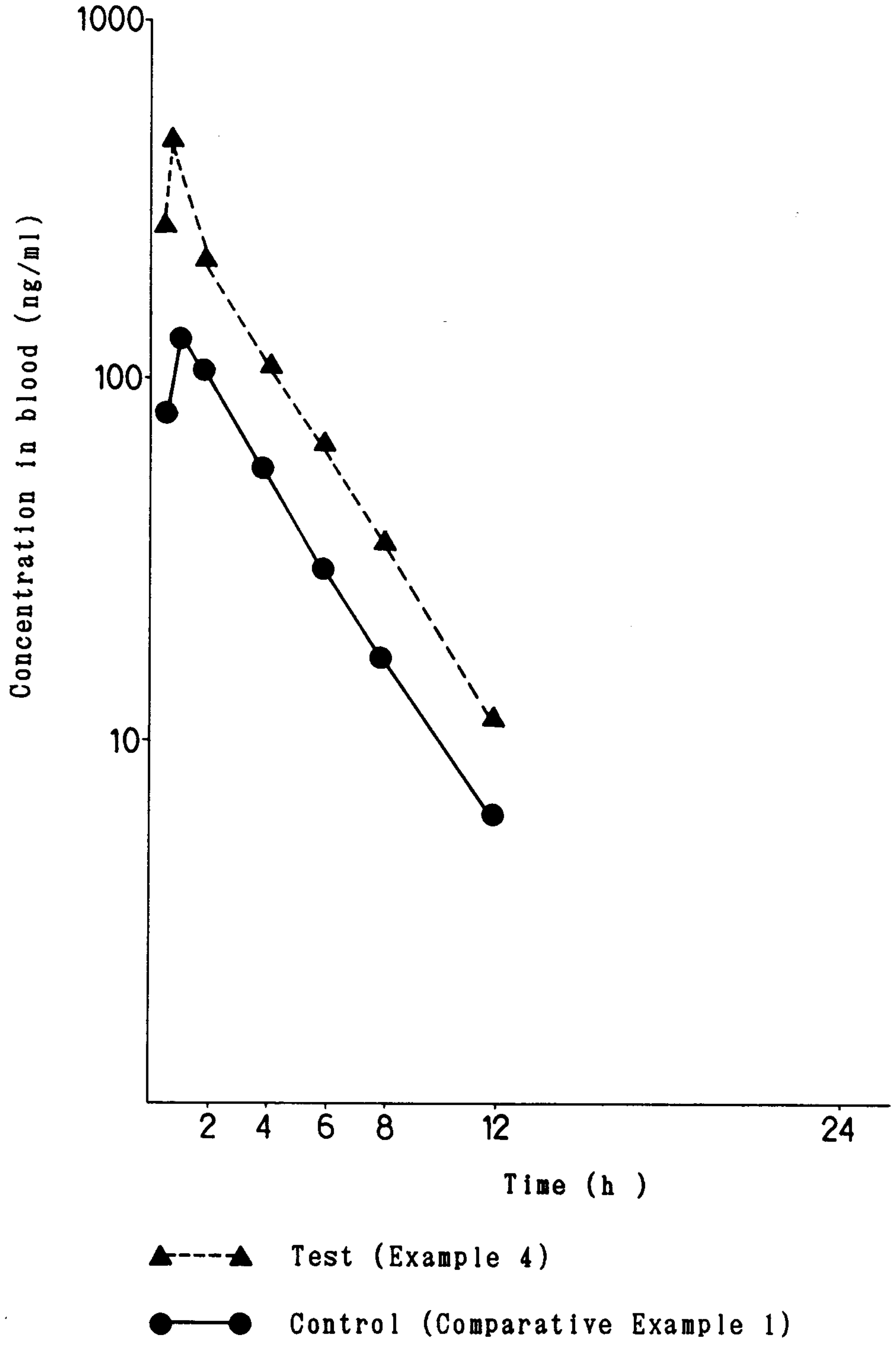
1000
100
10
Concentration in blood (ng/ml)
2
4
6
8
12
24
Time (h )
Test (Example 4)
Control (Comparative Example 1)

FIG. 2

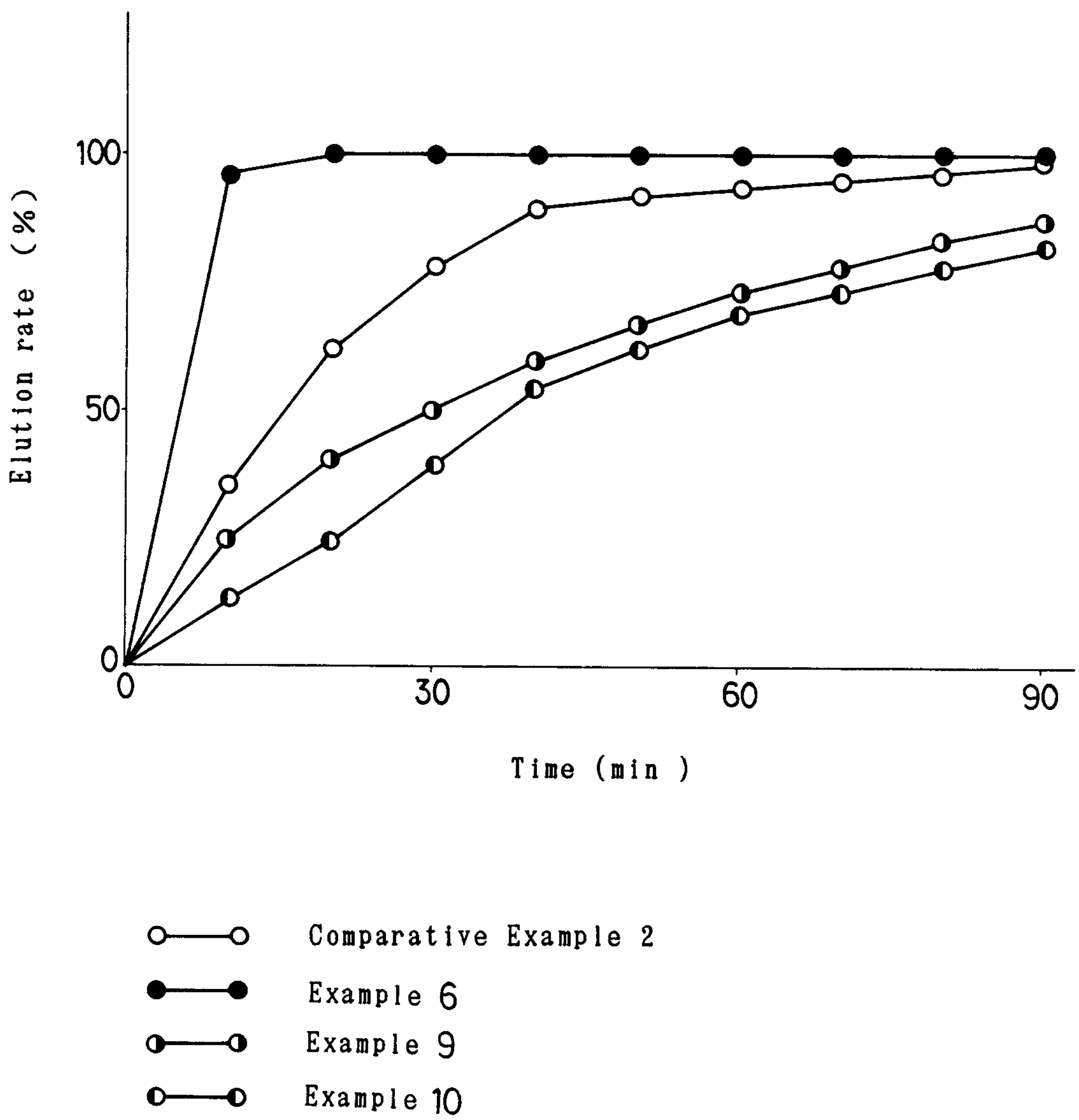
Elution rate (%)
100
50
0
0
30
60
90
Time (min )
Comparative Example 2
Example 6
Example 9
Example 10

FIG. 3

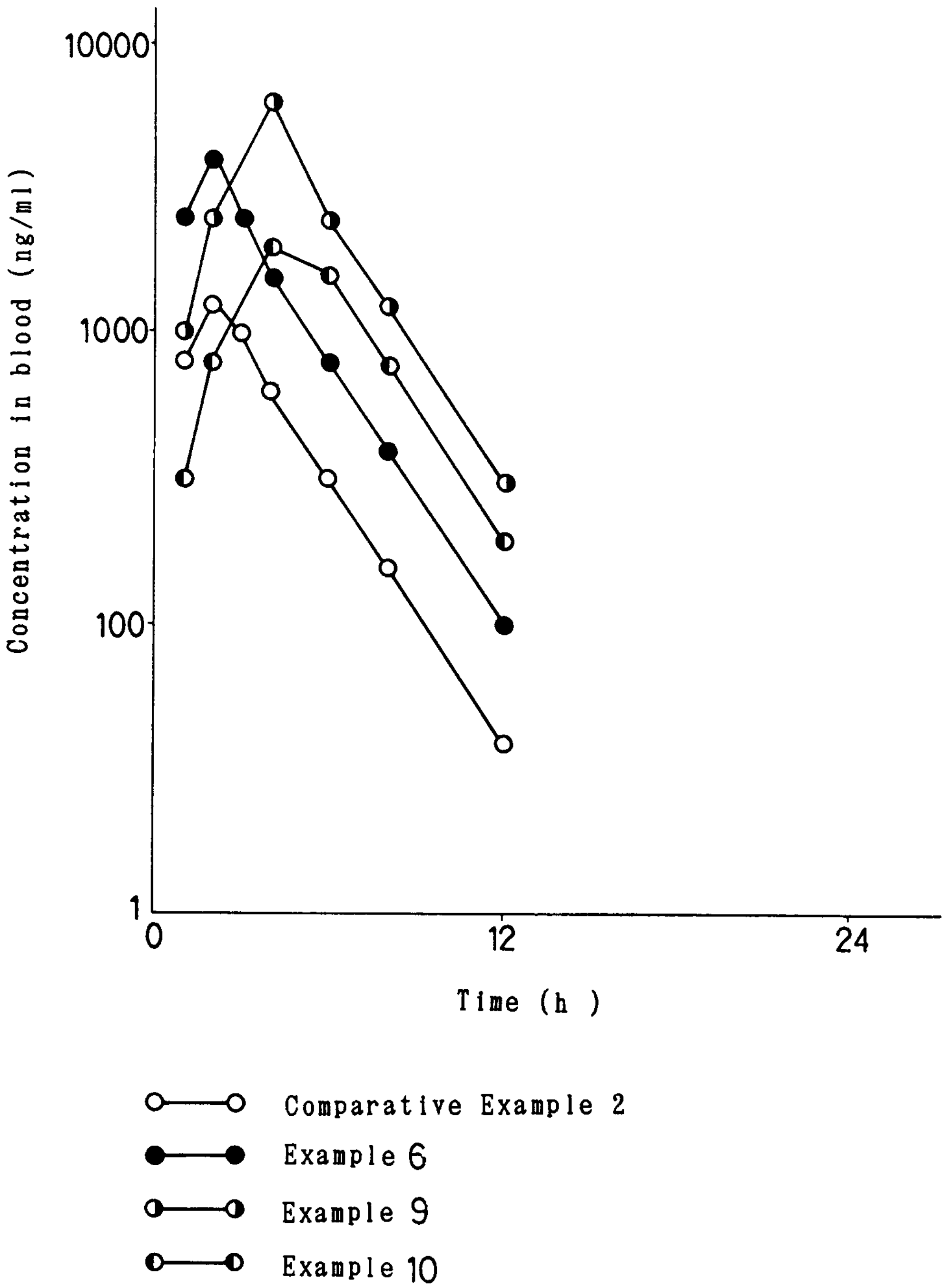
10000
1000
100
1
Concentration in blood (ng/ml)
0
12
24
Time (h )
Comparative Example 2
Example 6
Example 9
Example 10